(19) Europäisches Patentamt / European Patent Office / Office européen des brevets

(11) **EP 4 403 188 A1**

(12) **EUROPEAN PATENT APPLICATION**
published in accordance with Art. 153(4) EPC


(43) Date of publication:
**24.07.2024 Bulletin 2024/30**

(21) Application number: **21957067.8**

(22) Date of filing: **16.09.2021**

(51) International Patent Classification (IPC):
***A61K 47/68*** *(2017.01)* ***A61K 45/00*** *(2006.01)*
***A61P 35/00*** *(2006.01)*

(52) Cooperative Patent Classification (CPC):
**A61K 45/00; A61K 47/68; A61P 35/00**

(86) International application number:
**PCT/CN2021/118656**

(87) International publication number:
**WO 2023/039778 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Shanghai Miracogen Inc.**
**Shanghai 201203 (CN)**

(72) Inventors:
- **XIAO, Lili**
  **Shanghai 201203 (CN)**
- **HU, Chaohong**
  **Shanghai 201203 (CN)**

(74) Representative: **E. Blum & Co. AG**
**Franklinturm**
**Hofwiesenstrasse 349**
**8050 Zürich (CH)**


Remarks:
The complete document including Reference Table(s) and the Sequence Listing(s) can be downloaded from the EPO website

(54) **ANTIBODY DRUG CONJUGATE FORMULATION AND USE THEREOF**


(57) The present invention provides an antibody drug conjugate formulation, comprising an antibody drug conjugate or a salt thereof, a citric acid buffer solution, trehalose, sodium chloride and polysorbate 80. The antibody drug conjugate has a structure represented by $Ab\text{-}(L\text{-}D)_p$, wherein Ab represents an anti-epidermal growth factor receptor antibody, L represents a linker, and D represents a cytotoxic agent.


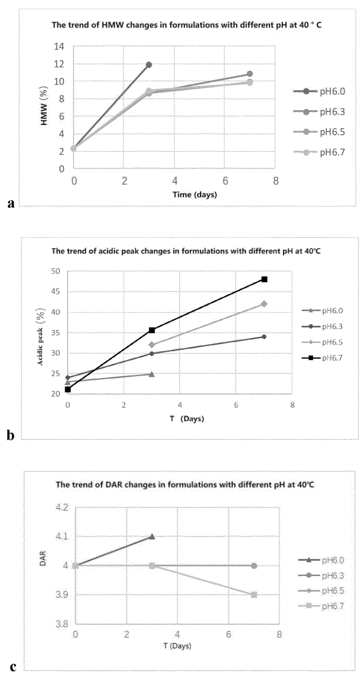

Fig. 10

EP 4 403 188 A1

## Description

### Field of the Invention

The present invention relates to the field of biomedicine, in particular to an antibody-drug conjugate formulation and use thereof.

### Background of the Invention

Epidermal growth factor receptor (EGFR, also known as HER1, c-ErbB1) is a cell surface receptor of epidermal growth factor family, is a transmembrane glycoprotein composed of 1186 amino acid residues, and has a molecular weight of 170 kD. EGFR belongs to type I tyrosine kinase receptor subfamily ErbB (ErbB 1-4) and has tyrosine kinase activity. EGFR is stably expressed in many epithelial tissues, including the skin and hair follicles. Abnormal expression of epidermal growth factor receptor or activation caused by receptor mutation may lead to carcinogenesis. There are many solid tumors where over expression of epidermal growth factor receptor are found, such as colorectal cancer, head-neck cancer, lung cancer, ovarian cancer, cervical cancer, bladder cancer and esophageal cancer. Growth factors such as transforming growth factor $\alpha$ and epidermal growth factor are endogenous ligands for EGFR. These ligands bind to epidermal growth factor receptor and activate intracellular tyrosine protein kinase activity, initiate a lot of downstream signal transduction pathways, thereby regulating growth and differentiation of normal cells, enhancing invasiveness of tumor cells, promoting angiogenesis and inhibiting apoptosis of tumor cells. Epidermal growth factor receptor overexpression in tumor and its important roles in the growth and differentiation of tumor cells make epidermal growth factor receptor a promising target for tumor therapy.

At present, there are two anti-epidermal growth factor receptor antibodies in the market, one is human-mouse chimeric antibody C225 antibody (Erbitux or Cetuximab, ImClone Company (now Eli Lilly Company)), which has a specific binding affinity to epidermal growth factor receptor, can block the binding between a ligand such as EGF or TGF and a epidermal growth factor receptor, inhibit its phosphorylation and downstream signal transduction, thereby inhibiting tumor cell growth, inducing apoptosis, reducing production of matrix metalloproteinases and vascular endothelial growth factor. The FDA of the United States approved the use of Erbitux for treatment of colorectal cancer in 2004, for treatment of head and neck cancer in 2006, and for other cancer indications in more clinical trials now. Clinically, the overall response rate (ORR) of the combination of Erbitux and irinotecan in treatment of colorectal cancer is 23%, and the ORR of the combination of Erbitux and chemotherapy drug such as fluoropyrimidine in treatment of head and neck cancer is 13%-30%. Because of being a human-mouse chimeric antibody, Erbitux induced an anti-therapeutic antibody response in 3.7% of the patients in the clinical trial.

Another anti-epidermal growth factor receptor antibody is panitumumab (Vectibix, Amgen Company), which is a fully humanized monoclonal antibody prepared by using transgenic mouse technology, and is free of mouse original protein sequence. The antibody targets epidermal growth factor receptor (EGFR), and was approved by the FDA in September 2006, used in combination with fluoropyrimidine, Oxaliplatin and Irinotecan or for treatment of EGFR positive metastatic colorectal cancer after chemotherapy. In 2006, FDA approved its monotherapy for the treatment of metastatic colorectal cancer (mCRC) with chemotherapy tolerance. However, panitumumab is an IgG2 subtype antibody, and compared with IgG1, IgG2 exhibits significantly decreased biological activities such as CDC activity and ADCC activity; in addition, IgG2 subtype antibodies usually have poor stability. These may be the main reasons that fully humanized antibody panitumumab shows no obvious advantages in clinical effects in comparison with chimeric antibody Erbitux. The overall survival rate (OR) in clinical treatment of colorectal cancer was merely 8% and the progression free survival was only extended by 3.6 months.

At present, large amount of clinical data showed that Erbitux and panitumumab had therapeutic effects only on wild type KRAS (KRAS wild type) with expression of EGFR, but had no tumor growth inhibitory activity to KRAS mutants. Therefore, the Guidelines published by the American Society of Clinical Oncology explicitly point out that anti-EGFR monoclonal antibody drugs are only applicable to KRAS wild type colorectal cancer patients (Allegra CJ, Jessup JM, Somerield MR, Hamilton SR, Hammond EH, Hayes DF, et al. American Society of Clinical Oncology provisional clinical opinion: testing for KRAS gene mutations in patients with metastatic colorectal carcinoma to predict response to anti-epidermal growth factor monoclonal antibody therapy. J. Clin Oncol. 2009; 27:2091-2096; Bardelfi A, Siena S. Molecular mechanisms of resistance to cetuximab and panitumumab in colorectal cancer. J Clin Oncol. 2010; 28:1254-1261).

Therefore, it is in need in the art to have humanized anti-epidermal growth factor receptor antibody drugs with biological activity, especially antibody drugs, such as antibody-drug conjugates, with curative effects to KRAS mutants, so as to further improve therapeutic efficacy and reduce side effects.

## Summary of the Invention

Through extensive experiments and creative efforts, the inventors of the present application provide an anti-EGFR antibody-drug conjugate formulation which has a good therapeutic effect on diseases related to EGFR, and compared with the prior art, the stability of the formulation is significantly improved.

In view of this, in the first aspect of the present invention, the present invention provides an antibody-drug conjugate formulation, comprising: an antibody-drug conjugate or a salt thereof with a concentration of 1-20 mg/mL (such as 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL or 20 mg/mL, or 3-5 mg/mL, or 2-6 mg/mL, or 1-7 mg/mL, or 1-8 mg/mL, or 1-9 mg/mL, or 1-10 mg/mL);

a citrate buffer with a concentration of 10-50 mM (such as 10 mM, 15 mM, 16 mM, 17 mM, 18 mM, 19 mM, 20 mM, 21 mM, 22 mM, 23 mM, 24 mM, 25 mM, 30 mM, 35 mM, 40 mM, 45 mM or 50 mM, or 19-21 mM, or 18-22 mM, or 17-23 mM, or 16-24 mM) and pH of 6.3-6.7 (such as 6.3, 6.4, 6.5, 6.6 or 6.7);
trehalose with a concentration of 1-10% (such as 1%, 2%, 3%, 4%, 4.5%, 5%, 5.5%, 6%, 6.5%, 7%, 8%, 9% or 10%, or 4-7%, or 4.5-6.5%, or 5-6%);
NaCl with a concentration of 0.1-2% (such as 0.1%, 0.2%, 0.3%, 0.4%, 0.5%, 0.6%, 0.7%, 0.8%, 0.9%, 1%, 1.5% or 2%, or 0.2-0.4%); and
Tween 80 with a concentration of 0.01-0.2% (such as 0.01%, 0.02%, 0.03%, 0.04%, 0.05%, 0.06%, 0.07%, 0.08%, 0.09%, 0.1%, 0.15% or 0.2%, or 0.02-0.08%, or 0.03-0.07%, or 0.04-0.06%);
wherein the antibody-drug conjugate has a structure of formula I,

$$\text{Ab-(L-D)}_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID Nos: 5-7 or mutants thereof, and CDR1, CDR2, and CDR3 in a light chain variable region respectively comprise sequences as shown in SEQ ID Nos: 12-14 or mutants thereof;
L represents a linker, and the linker is 6-maleimidocaproyl-valine-citrulline-p-aminobenzyloxycarbonyl (MC-vc-PAB);
D represents a cytotoxic agent, and the cytotoxic agent is MMAE; and
p represents 1-9 (such as 1, 2, 3, 4, 5, 6, 7, 8 or 9, or such as 1-7), such as 2-6, 3-5, specifically, such as 3.9, 4.0 or 4.1.

It should be noted that the concentration of trehalose, NaCl or Tween 80 represents the mass volume concentration (w/v%), which refers to the mass (unit: g) of trehalose, sodium chloride or Tween 80 per 100 mL of the antibody-drug conjugate formulation.

In addition, "citrate buffer" is prepared from citric acid and sodium citrate, and its different pH values are achieved by adjusting different ratios of citric acid to sodium citrate. The concentration of the "citrate buffer" refers to the total concentration of citric acid and sodium citrate.

In addition, it should be noted that the "antibody-drug conjugate" described above refers to a composition comprising ADC molecules with the same or different DAR.

Specifically, the present invention provides a composition comprising a plurality of anti-EGFR ADC molecules. In some cases, each ADC in the composition described herein comprises the same number of one or more cytotoxic agents. In other cases, each ADC in the composition described herein comprises a different number of one or more cytotoxic agents.

In the antibody-drug conjugate described herein, each anti-EGFR antibody can be conjugated with 1, 2, 3, 4, 5, 6, 7, 8, or more cytotoxic agents.

The drug-to-antibody ratio (DAR) mentioned above refers to the number of molecules of cytotoxic agent conjugated to anti-EGFR antibodies. The number of molecules of cytotoxic agent contained in the ADC described herein is generally an integer, when the number (e.g., p in formula I) of molecules of cytotoxic agent contained in the ADC described herein is a fraction, that fraction refers to the average number of cytotoxic agents conjugated to each anti-EGFR antibody in a composition comprising a plurality of ADC molecules.

In some embodiments, the concentration of the antibody-drug conjugate or a salt thereof is 2 mg/mL-6 mg/mL.

In some embodiments, the concentration of the antibody-drug conjugate or a salt thereof is 4 mg/mL.

In some embodiments, the concentration of the citrate buffer is 15 mM-25 mM.

In some embodiments, the concentration of the citrate buffer is 20 mM.

In some embodiments, the pH of the citrate buffer is 6.3-6.5.

In some embodiments, the pH of the citrate buffer is 6.3.

In some embodiments, the concentration of the trehalose is 4%-7%.

In some embodiments, the concentration of the trehalose is 5.5%.

In some embodiments, the concentration of the NaCl is 0.1%-0.5%.

In some embodiments, the concentration of the NaCl is 0.3%.

In some embodiments, the concentration of the Tween 80 is 0.01%-0.1%.

In some embodiments, the concentration of the Tween 80 is 0.05%.

In some embodiments, FR1, FR2, FR3, and FR4 regions in the heavy chain variable region of the anti-EGFR antibody respectively comprise sequences as shown in SEQ ID Nos: 8-11 or mutants thereof.

In some embodiments, FR1, FR2, FR3, and FR4 regions in the light chain variable region of the anti-EGFR antibody respectively comprise sequences as shown in SEQ ID Nos: 15-18 or mutants thereof.

In some embodiments, the heavy chain constant region of the anti-EGFR antibody is selected from human IgG, IgM, IgA, IgD, and IgA constant regions or mutants thereof.

In some embodiments, the IgG is selected from IgG1, IgG2, IgG3 and IgG4.

In some embodiments, the light chain constant region of the anti-EGFR antibody is selected from human lambda and kappa constant regions or mutants thereof.

In some embodiments, the sequence of the heavy chain variable region of the anti-EGFR antibody comprises a sequence as shown in SEQ ID NO: 1, or a sequence having greater than 70%, preferably greater than 75%, 80%, 85%, 90%, 95%, or 99% identity to the sequence as shown in SEQ ID NO: 1.

In some embodiments, the sequence of the heavy chain variable region of the anti-EGFR antibody is shown in SEQ ID NO: 1.

In some embodiments, the sequence of the light chain variable region of the anti-EGFR antibody comprises a sequence as shown in SEQ ID NO: 2, or a sequence having greater than 70%, preferably greater than 75%, 80%, 85%, 90%, 95%, or 99% identity to the sequence as shown in SEQ ID NO: 2.

In some embodiments, the sequence of the light chain variable region of the anti-EGFR antibody is shown in SEQ ID NO: 2.

In some embodiments, the sequence of the heavy chain constant region of the anti-EGFR antibody comprises a sequence as shown in SEQ ID NO: 3, or a sequence having greater than 70%, preferably greater than 75%, 80%, 85%, 90%, 95%, or 99% identity to the sequence as shown in SEQ ID NO: 3.

In some embodiments, the sequence of the heavy chain constant region of the anti-EGFR antibody is shown in SEQ ID NO: 3.

In some embodiments, the sequence of the light chain constant region of the anti-EGFR antibody comprises a sequence as shown in SEQ ID NO: 4, or a sequence having greater than 70%, preferably greater than 75%, 80%, 85%, 90%, 95%, or 99% identity to the sequence as shown in SEQ ID NO: 4.

In some embodiments, the sequence of the light chain constant region of the anti-EGFR antibody is shown in SEQ ID NO: 4.

In some embodiments, the trehalose is trehalose dihydrate.

In some embodiments, the citrate buffer is prepared from citric acid and sodium citrate.

In some embodiments, the citric acid is citric acid monohydrate.

In some embodiments, the sodium citrate is sodium citrate dihydrate.

In the second aspect of the present invention, the present invention provides a method for preparing the formulation described above, comprising:

1) allowing the anti-EGFR antibody as described above to have a reduction reaction with a reducing agent to obtain a reduced anti-EGFR antibody;
2) allowing the reduced anti-EGFR antibody to have a conjugation reaction with vcMMAE; and
3) quenching the conjugation reaction, and performing buffer exchange of the conjugation reaction product to obtain the antibody-drug conjugate formulation as described above.

In some embodiments, the method includes:

(1) exchanging the anti-EGFR antibody described above (preferably 10 mg) (preferably three times) into a reducing buffer (preferably comprising: 25 mM sodium borate, pH 8.0, 25 mM NaCl, 5 mM EDTA), detecting the concentration of the protein, and calculating the amount of the protein;
(2) adding DTT to the product of step (1) to have a reaction at room temperature for 1-5 h (preferably 2 h) to obtain a reduced anti-EGFR antibody, wherein the amount of substance of the DTT is 2.0-3.0 times, preferably 2.5 times,

the amount of substance of the protein;
(3) exchanging the product of step (2) (preferably three times) into a coupling buffer (preferably comprising: 50 mM Tris, pH7.2, 150 mM NaCl, 5 mM EDTA), and calculating the amount of substance of free thiol groups;
(4) adding vc-MMAE (i.e., MC-vc-PAB-MMAE) to the product of step (3) to have a reaction at room temperature for 1-5 h (preferably 2 h), wherein the amount of substance of the vc-MMAE is 1.0-1.5 times, preferably 1.1 times, the amount of substance of the reduced anti-EGFR antibody;
(5) adding N-acetylcysteine to the product of step (4) and allowing to stand (preferably for 5 min) to obtain a mixed solution containing the antibody-drug conjugate described above, wherein the amount of substance of the N-acetylcysteine is 15-25 times, preferably 20 times, the amount of substance of the vc-MMAE; and
(6) exchanging the mixed solution of step (5) (preferably three times) into the conjugation stock solution to obtain the antibody-drug conjugate formulation;

the conjugation stock solution comprises:

a citrate buffer with a concentration of 10-50 mM (such as 10 mM, 15 mM, 16 mM, 17 mM, 18 mM, 19 mM, 20 mM, 21 mM, 22 mM, 23 mM, 24 mM, 25 mM, 30 mM, 35 mM, 40 mM, 45 mM or 50 mM, or 19-21 mM, or 18-22 mM, or 17-23 mM, or 16-24 mM) and pH of 6.3-6.7 (such as 6.3, 6.4, 6.5, 6.6 or 6.7);
trehalose with a concentration of 1-10% (such as 1%, 2%, 3%, 4%, 4.5%, 5%, 5.5%, 6%, 6.5%, 7%, 8%, 9% or 10%, or 4-7%, or 4.5-6.5%, or 5-6%);
NaCl with a concentration of 0.1-2% (such as 0.1%, 0.2%, 0.3%, 0.4%, 0.5%, 0.6%, 0.7%, 0.8%, 0.9%, 1%, 1.5% or 2%, or 0.2-0.4%); and
Tween 80 with a concentration of 0.01-0.2% (such as 0.01%, 0.02%, 0.03%, 0.04%, 0.05%, 0.06%, 0.07%, 0.08%, 0.09%, 0.1%, 0.15% or 0.2%, or 0.02-0.08%, or 0.03-0.07%, or 0.04-0.06%);
in the antibody-drug conjugate formulation, the concentration of the antibody-drug conjugate is 1-20 mg/mL (such as 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL or 20 mg/mL, or 3-5 mg/mL, or 2-6 mg/mL, or 1-7 mg/mL, or 1-8 mg/mL, or 1-9 mg/mL, or 1-10 mg/mL).

In some embodiments, the concentration of the citrate buffer is 15 mM-25 mM.
In some embodiments, the concentration of the citrate buffer is 20 mM.
In some embodiments, the pH of the citrate buffer is 6.3-6.5.
In some embodiments, the pH of the citrate buffer is 6.3.
In some embodiments, the concentration of the trehalose is 4%-7%.
In some embodiments, the concentration of the trehalose is 5.5%.
In some embodiments, the concentration of the NaCl is 0.1%-0.5%.
In some embodiments, the concentration of the NaCl is 0.3%.
In some embodiments, the concentration of the Tween 80 is 0.01%-0.1%.
In some embodiments, the concentration of the Tween 80 is 0.05%.
In some embodiments, the concentration of the antibody-drug conjugate is 2 mg/mL-6 mg/mL.
In some embodiments, the concentration of the antibody-drug conjugate is 4 mg/mL.
In some embodiments, the trehalose is trehalose dihydrate.
In some embodiments, the citrate buffer is prepared from citric acid and sodium citrate.
In some embodiments, the citric acid is citric acid monohydrate.
In some embodiments, the sodium citrate is sodium citrate dihydrate.
In the third aspect of the present invention, the present invention provides an antibody-drug conjugate formulation prepared by the method described above.
In the fourth aspect of the present invention, the present invention provides a composition comprising the formulation described above.
In some embodiments, the composition further comprises a known chemotherapeutic drug for the treatment of a tumor, and the chemotherapeutic drug can be, for example, doxorubicin (Adriamycin), cyclophosphamide and taxanes [e.g., paclitaxel (Taxol) and docetaxel (Taxotere)], capecitabine (Xeloda), gemcitabine (Gemzar), vinorelbine (Navelbine), tamoxifen, aromatase inhibitor (Arimidex, Femara, Aromasin), 5-FU/leucovorin, irinotecan (camptosar), oxaliplatin, cisplatin, carboplatin, estramustine, mitoxantrone (Novantrone), prednisone, vincristine (Oncovin), Doxorubicin, prednisone, etc., or a combination thereof.
In some embodiments, the composition further comprises a known immunotherapeutic drug for treating a tumor, wherein the immunotherapeutic drug is, for example, a PD-1 monoclonal antibody (such as Pembrolizumab and Nivolumab), a PD-L1 monoclonal antibody (such as Atezolizumab), a TIGIT monoclonal antibody, a 4-1BB monoclonal antibody, a VEGFR2 monoclonal antibody (such as Ramucirumab and Apatinib), an HER2 monoclonal antibody (such as Trastuzumab, Trastuzumab biosimilar, and Trastuzumab-dkst), etc., or their combination.

In some embodiments, the composition further comprises an immunosuppressive agent selected from: (1) glucocorticoid, such as cortisone and prednisone; (2) microbial metabolite, such as cyclosporine and tacrolimus, etc.; (3) anti-metabolite, such as azathioprine and 6-mercaptopurine, etc.; (4) polyclonal and monoclonal anti-lymphocyte antibodies, such as anti-lymphocyte globulin and OKT3, etc.; (5) alkylating agent, such as cyclophosphamide. Specifically, the immunosuppressive agent is, for example, methylprednisolone, prednisone, azathioprine, Prograf, Zenapax, Simulect, cyclosporine, tacrolimus, rapamycin, mycophenolate, mizoribine, cyclophosphamide, Fingolimod, etc.

In some embodiments, the composition further comprises a pharmaceutically acceptable carrier, a diluent or an excipient.

In the fifth aspect of the present invention, the present invention provides use of the formulation described above or the composition described above in the preparation of a medicament for preventing and/or treating an EGFR-related disease.

In the sixth aspect of the present invention, the present invention provides the formulation described above or the composition described above for use in preventing and/or treating an EGFR-related disease.

In the seventh aspect of the present invention, the present invention provides a method for preventing and/or treating an EGFR-related disease, comprising: administering to a subject in need thereof a prophylactically and/or therapeutically effective amount of the formulation described above or the composition described above.

In some embodiments, the EGFR-related disease is an EGFR-related tumor, such as a tumor related to EGFR overexpression, further, for example, selected from the group consisting of colon cancer, rectal cancer, head and neck cancer, lung cancer, ovarian cancer, cervical cancer, bladder cancer, esophageal cancer, breast cancer, kidney cancer, prostate cancer, stomach cancer, pancreatic cancer and brain glioma.

In some embodiments, the tumor is a tumor with KRAS gene mutation, further, for example, colon cancer, rectal cancer, lung cancer or pancreatic cancer with KRAS gene mutation.

In some embodiments, the tumor is a tumor with BRAF gene mutation, further, for example, selected from the group consisting of colon cancer, rectal cancer and lung cancer with BRAF gene mutation.

In the eighth aspect of the present invention, the present invention provides use of the formulation described above or the composition described above in the preparation of a reagent or a medicament for inhibiting tumor angiogenesis, delaying tumor progression, inhibiting tumor growth, or inhibiting tumor cell proliferation.

In the ninth aspect of the present invention, the present invention provides the formulation described above or the composition described above for use in inhibiting tumor angiogenesis, delaying tumor progression, inhibiting tumor growth, or inhibiting tumor cell proliferation.

In the tenth aspect of the present invention, the present invention provides a method for inhibiting tumor angiogenesis, delaying tumor progression, inhibiting tumor growth, or inhibiting tumor cell proliferation, comprising: administering to a subject in need thereof an effective amount of the formulation described above or the composition described above.

In some embodiments, the tumor is selected from the group consisting of colon cancer, rectal cancer, head and neck cancer, lung cancer, ovarian cancer, cervical cancer, bladder cancer, esophageal cancer, breast cancer, kidney cancer, prostate cancer, stomach cancer, pancreatic cancer and brain glioma.

In some embodiments, the tumor is a tumor with KRAS gene mutation, further, for example, colon cancer, rectal cancer, lung cancer or pancreatic cancer with KRAS gene mutation.

In some embodiments, the tumor is a tumor with BRAF gene mutation, further, for example, selected from the group consisting of colon cancer, rectal cancer and lung cancer with BRAF gene mutation.

Beneficial Effect

The antibody-drug conjugate formulation of the present invention remains clear in appearance under different temperature conditions, its DAR remains basically unchanged, its amount of HMW% and acidic peak % is moderate, and its formulation stability is significantly improved as compared with that in the prior art.

## Brief Description of the Drawings

Fig. 1 is a HIC-HPLC chromatogram for determining the drug/antibody ratio of an antibody drug conjugate.
Fig. 2 shows results of detection of inhibition activity on cells in vitro of monoclonal antibody and antibody-drug conjugate, where ◦ represents the monoclonal antibody BA03 and ▲ represents the antibody-drug conjugate MYK-3.
Fig. 3 shows the growth inhibition activity of MYK-3 against colon cancer cell HT-29, where ◦ represents the monoclonal antibody BA03 and ▲ represents the antibody-drug conjugate MYK-3.

Fig. 4 shows the growth inhibition activity of MYK-3 against brain glioma cancer cell U87-MG, where ◦ represents the monoclonal antibody BA03 and ▲ represents the antibody-drug conjugate MYK-3.

Fig. 5 shows the growth inhibition activity of MYK-3 against lung cancer cell A549, where ◦ represents the monoclonal antibody BA03 and ▲ represents the antibody-drug conjugate MYK-3.

Fig. 6 shows the growth inhibition activity of MYK-3 against KRAS mutant colon cancer cell LoVo, where ◇ represents the monoclonal antibody Erbitux and ▲ represents the antibody-drug conjugate MYK-3.

Fig. 7 shows effects of monoclonal antibody and antibody-drug conjugates on volume of HT-29 colon cancer xenografted tumor in mice, in which the data are expressed as the mean ± standard deviation; * indicates P<0.05, ** indicates P<0.01, and *** indicates P<0.001, as compared with the buffer control group.

Fig. 8 shows effects of monoclonal antibody and antibody-drug conjugate on body weight of mice of HT-29 colon cancer xenograft model.

Fig. 9 shows the growth inhibition activity of MYK-3 on xenografted tumor with KRAS mutant colon cancer cell LoVo in nude mice.

Fig. 10 shows the comparison of changes in quality attributes of MYK-3 in citrate buffers of different pH.

## Detailed Description of the Embodiments

The embodiments of the disclosure will be described in detail below in conjunction with examples. However, those skilled in the art will understand that the following examples are only used to illustrate the invention, not to limit the scope of the invention. Those without specific conditions in the examples are generally implemented under conventional conditions or conditions recommended by the manufacturers. The reagents or instruments used without specifying the manufacturers are all conventional products that can be purchased commercially.

In the invention, all scientific and technical terms used herein have the meanings commonly understood by those skilled in the art unless specified otherwise. In addition, the related terms of protein and nucleic acid chemistry, molecular biology, cell and tissue culture, microbiology, immunology, and laboratory procedures used herein are all terms and routine procedures widely used in the corresponding art. Moreover, definitions and explanations of related terms are provided below to understand the invention better.

Unless otherwise stated, in the invention, any concentration range, percentage range, ratio range or numerical range shall be understood to include any integer value within the stated range and, where appropriate, fractional values within the stated range.

The term "antibody", as used herein, refers to an immunoglobulin molecule usually composed of two pairs of identical polypeptide chains, each pair having a "light" (L) chain and a "heavy" (H) chain. The light chains of the antibody can be divided into two categories: $\kappa$ and $\lambda$. The heavy chains can be divided into five categories: $\mu$, $\delta$, $\gamma$, $\alpha$ or $\varepsilon$. According to the difference of heavy chains, antibodies can be divided into five categories: IgM, IgD, IgG, IgA and IgE. Within the light and heavy chains, the variable and constant regions are connected by a "J" region of approximately 12 or more amino acids, and the heavy chain also has a "D" region of approximately 3 or more amino acids. Each heavy chain consists of a heavy chain variable region ($V_H$) and a heavy chain constant region ($C_H$). The heavy chain constant region consists of three domains ($C_H1$, $C_H2$ and $C_H3$). Each light chain consists of a light chain variable region ($V_L$) and a light chain constant region ($C_L$). The light chain constant region consists of one domain, $C_L$. The constant regions of the antibody can mediate the binding of the immunoglobulin to host tissues or factors, including various cells of the immune system (e.g., effector cells) and component C1q of the complement system. $V_H$ and $V_L$ can also be subdivided into highly variable regions called complementarity-determining regions (CDRs), interspersed with more conservative regions called framework regions (FRs). Each $V_H$ and $V_L$ consists of, from the amino terminus to the carboxyl terminus, three CDRs and four FRs arranged in the following order: FR1, CDR1, FR2, CDR2, FR3, CDR3, FR4. The variable regions ($V_H$ and $V_L$) of each heavy chain/light chain pair form an antibody binding site respectively. The assignment of amino acids to each region or structural domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

The monoclonal antibody variant described herein can be obtained through traditional genetic engineering methods. Those skilled in the art are fully aware of methods for modifying DNA molecules using nucleic acid mutations. In addition, nucleic acid molecules encoding heavy chain and light chain variants can also be obtained through chemical synthesis.

In the present invention, algorithms used to determine sequence identity (homology) and percent sequence similarity are, for example, the BLAST and BLAST 2.0 algorithms, respectively described by Altschul et al., (1977) Nucl. Acid. Res. 25: 3389-3402 and Altschul et al., (1990) J. Mol. Biol. 215: 403-410. BLAST and BLAST2.0 can be used to determine percent of amino acid sequence identity of the present invention using, for example, parameters described in the references or default parameters. Software used to perform BLAST analysis is publicly available through the National Center for Biotechnology Information.

As described herein, said amino acid sequence having at least 70% identity to an amino acid sequence includes a polypeptide sequence that is substantially identical to said amino acid sequence, e.g., those sequences having at least 70%, preferably at least 75%, 80%, 85%, 86%, 87%, 88%, 89%, 90%, 91%, 92%, 93%, 94%, 95%, 96%, 97%, 98%, 99% or higher identity to the polypeptide sequence of the invention when using the methods described herein (such as BLAST analysis using standard parameters).

The mutant of said amino acid sequence, as used herein, refers to a sequence which has identity of more than 70%, such as more than 75%, 80%, 85%, 90%, 91%, 92%, 93%, 94%, 95%, 96%, 97%, 98%, or 99%, to said amino acid sequence, such as the sequence having three, two or one substitution, deletion or addition of amino acids. Preferably, no more than three amino acids are substituted, added or deleted. More preferably, no more than two amino acids are substituted, added or deleted. Most preferably, no more than one amino acid is substituted, added or deleted.

A "substitutional" variant is one in which at least one amino acid residue in the native sequence has been removed and a different amino acid inserted in its same position. The substitutions can be single, wherein only one amino acid is substituted in the molecule, or multiple, wherein the same molecule has two or more amino acids substituted. Multiple substitutions can be made at consecutive sites. Likewise, one amino acid may be substituted by multiple residues, wherein such variants include both substitutions and insertions. An "insertion" (or "additive") variant is one in which one or more amino acids are inserted into a particular position immediately adjacent to a native sequence. Immediately adjacent to an amino acid means attachment to the alpha-carboxyl or alpha-amino functional group of the amino acid. A "deletion" variant is one in which one or more amino acids in the native amino acid sequence have been removed. Typically, deletion variants have one or two amino acids deleted in a specific region of their molecule.

In the present invention, the structure of the MMAE is

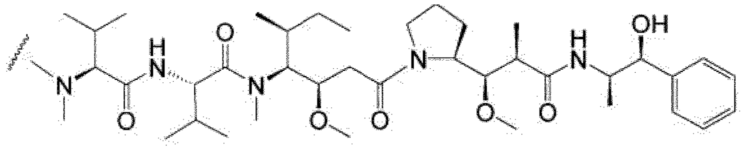

In the present invention, the L-D in formula I is MC-vc-PAB-MMAE having a structure represented by:

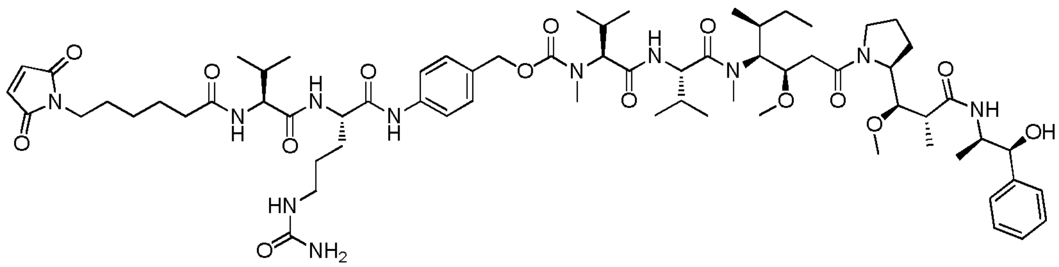

In the present invention, the anti-EGFR antibody Ab is linked to the L-D through thiol generated after its own disulfide bond is reduced, as shown below:

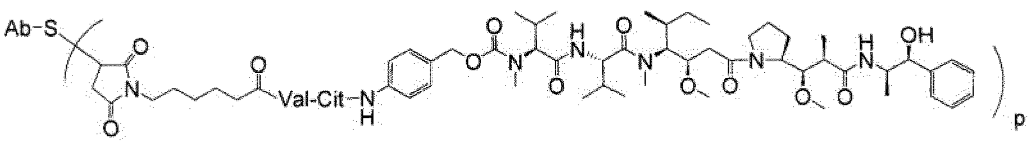

In the present invention, the drug antibody ratio (DAR) or drug load is represented by p, i.e., the average number of drug modules (i.e., cytotoxic agents) per antibody in the molecule of formula I: Ab-$(L-D)_p$, which may be an integer or a fraction. ADC of general formula I includes a collection of antibodies conjugated with a range of drug modules. The average number of drug modules per antibody in an ADC formulation from conjugation reaction can be verified by conventional means, such as mass spectrometry, ELISA, HIC and HPLC. The quantitative distribution of ADC in p can also be determined. In some cases, the separation, purification and verification of homogeneous ADC with p of a certain value from ADC with other DAR can be achieved by means such as reversed-phase HPLC or electrophoresis.

In certain embodiments, less than the theoretical maximum of the drug moiety is conjugated to the antibody in

the conjugation reaction. In general, antibodies do not contain many free and reactive cysteine thiol groups that can link drug moieties; in fact, most cysteine thiol groups in antibodies exist as disulfide bridges. In certain embodiments, the antibody can be reduced with a reducing agent such as dithiothreitol (DTT) or tris(2-carboxyethyl)phosphine (TCEP) under partially or fully reducing conditions to generate reactive cysteine thiol groups.

In the present invention, "treatment" refers to a clinical intervention that attempts to alter the natural course of the individual or cell being treated, either for prevention or in the course of clinical pathology. The desired effects of treatment include preventing the occurrence or recurrence of disease, alleviating symptoms, attenuating any direct or indirect pathological consequences of the disease, preventing metastasis, slowing down the disease progression, ameliorating or alleviating the disease state, and eliminating or improving prognosis. In some embodiments, the antibody-drug conjugate of the present invention is used to delay the onset of a disease or disorder, or to slow down the progression of a disease or disorder. The above-described parameters for assessing successful treatment and amelioration of disease can be readily measured by routine procedures familiar to physicians. For cancer treatment, efficacy can be measured, for example, by assessing time to progression of disease (TTP) and/or determining response rate (RR).

In the present invention, the term "subject" refers to vertebrates. In some embodiments, the vertebrates are mammals. The mammals include, but are not limited to, livestock (such as cattle), pets (such as cats, dogs, and horses), primates, mice and rats. In some embodiments, the mammals refer to humans.

In the present invention, "effective amount" refers to an amount effective to achieve the desired therapeutic or prophylactic effect at the necessary dose and time. The "therapeutically effective amount" of a substance/molecule of the present invention may vary depending on factors such as the disease state, age, sex and weight of the individual and the ability of the substance/molecule to elicit a desired response in the individual. A therapeutically effective amount also encompasses an amount in which any toxic or detrimental consequences of the substance/molecule are outweighed by the therapeutically beneficial effects. The "prophylactically effective amount" refers to an amount effective at the necessary dose and time to achieve the desired prophylactic effect. Usually, but not necessarily, the prophylactically effective amount will be less than the therapeutically effective amount because the prophylactic dose is administered to the subject prior to the onset of the disease or at an early stage of the disease. In the case of cancer, the therapeutically effective amount of the drug reduces the number of cancer cells; shrinks the tumor size; inhibits (i.e., slows to some extent, preferably stops) infiltration of cancer cells into surrounding organs; inhibits (i.e., slows to some extent, preferably stops) tumor metastasis; inhibits tumor growth in some degree; and/or alleviates one or more symptoms associated with cancer in some degree.

For the prevention or treatment of disease, the appropriate dosage of the antibody-drug conjugate of the present invention (when used alone or in combination with one or more other therapeutic agents such as chemotherapeutic agents) will depend on the type of disease to be treated, the type of the antibody-drug conjugate, severity and progression of the disease, whether the antibody-drug conjugate is administered for prophylactic or therapeutic purposes, previous therapies, the patient's clinical history and reactivity to the antibody-drug conjugate, and the attending physician's judgment. Suitably, the antibody-drug conjugate is administered to the patient either once or over a series of treatments.

"Pharmaceutically acceptable carrier", as used herein, generally includes pharmaceutically acceptable carriers, excipients or stabilizers that are nontoxic for cells or mammals to which they are exposed at the doses and concentrations employed. Typically, the physiologically acceptable carriers refer to aqueous pH buffer solutions. Examples of physiologically acceptable carriers include buffers, such as phosphates, citrates, and other organic acids; antioxidants, including ascorbic acid; low-molecular-weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, arginine or lysine; monosaccharides, disaccharides and other carbohydrates, including glucose, mannose, sucrose, trehalose or dextrin; chelating agents, such as EDTA; sugar alcohols, such as mannitol or sorbitol; salt-forming counterions, such as sodium; and/or nonionic surfactants, such as TWEEN™, polyethylene glycol (PEG) and PLURONICS™.

In some embodiments, the pharmaceutically acceptable salt is an inorganic acid salt or an organic acid salt, wherein the inorganic acid salt is hydrochloride, hydrobromide, hydroiodide, nitrate, bicarbonate, carbonate, sulfate or phosphate, the organic acid salt is formate, acetate, propionate, benzoate, maleate, fumarate, succinate, tartrate, citrate, ascorbate, $\alpha$-ketoglutarate, $\alpha$-glycerophosphate, alkyl sulfonate or aryl sulfonate; preferably, the alkyl sulfonate is methane sulfonate or ethanesulfonate; the aryl sulfonate is benzene sulfonate or p-toluenesulfonate.

Pharmaceutically acceptable salts can be obtained using standard procedures well known in the field, for example, by reacting a sufficient amount of a basic compound with a suitable acid which provides a pharmaceutically acceptable anion.

In the present invention, KRAS gene has the same meaning as K-RAS gene. It is a member of the RAS gene family, encoding K-ras protein, and is related to the generation, proliferation, migration, diffusion and angiogenesis of various tumors. Its common mutation sites are codons 12 and 13 of exon 2 of the K-RAS gene, and codon 61 of exon 3. There are 7 mutation hotspots: G12C, G12R, G12S, G12V, G12D, G12A, and G13V/D. These 7 mutations account for more than 90%. In one embodiment of the present invention, the tumor is a tumor with KRAS gene mutation related

to EGFR overexpression.

In the present invention, the BRAF (v-raf murine sarcoma viral oncogene homologB1) gene is a proto-oncogene and a member of the RAF family. About 8% of human tumors have BRAF mutations, and most of the BRAF gene mutations are in the form of BRAFV600E mutations. This mutation leads to the continuous activation of the downstream MEK/ERK signaling pathway, which is crucial for tumor growth, proliferation, invasion and metastasis. In one embodiment of the present invention, the tumor is a tumor with BRAF gene mutation related to EGFR overexpression.

In the present invention, the 20 conventional amino acids and their abbreviations follow conventional usage. See Immunology-A Synthesis (second version, E.S. Golub and D.R. Gren, Eds., Sinauer Associates, Sunderland, Mass. (1991)), which is incorporated herein by reference.

The antibody BA03 in the present invention is the BA03 in the Chinese invention patent application CN103772504A. For its preparation method, refer to Example 3 in the patent application. The sequences of each part of the antibody are as follows:
the sequence of the heavy chain variable region is:

QVQLQESGPGLVKPSETLSLTCTVSGFSLSNYDVHWVRQAPGKGLEWLGVIWSGGNTDYNTPFTSR
LTISVDTSKNQFSLKLSSVTAADTAVYYCARALDYYDYEFAYWGQGTLVTVSS (SEQ ID NO: 1).

In the sequence, the underlined parts are CDR1 (SEQ ID NO: 5), CDR2 (SEQ ID NO: 6), and CDR3 (SEQ ID NO: 7), respectively;
the parts without underline are FR1 (SEQ ID NO: 8), FR2 (SEQ ID NO: 9), FR3 (SEQ ID NO: 10), and FR4 (SEQ ID NO: 11), respectively.

The sequence of the light chain variable region is:

EIVLTQSPDFQSVTPKEKVTITCRASQSIGTNIHWYQQKPDQSPKLLIKYASESISGIPSRFSGSGSGTD
FTLTINSLEAEDAATYYCQQNNEWPTSFGQGTKLEIK (SEQ ID NO: 2).

In the sequence, the underlined parts are CDR1 (SEQ ID NO: 12), CDR2 (SEQ ID NO: 13), and CDR3 (SEQ ID NO: 14), respectively;
the parts without underline are FR1 (SEQ ID NO: 15), FR2 (SEQ ID NO: 16), FR3 (SEQ ID NO: 17), and FR4 (SEQ ID NO: 18), respectively.

The sequence of the heavy chain constant region is:

ASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLSPGK
(SEQ ID NO: 3).

The sequence of the light chain constant region is:

RTVAAPSVFIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 4).

The present invention will be further described below with reference to specific embodiments.

Example 1: Preparation method of antibody-drug conjugate

10 mg of antibody BA03 was buffer exchanged into a reducing buffer (25 mM sodium borate, pH 8.0, 25 mM NaCl, 5 mM EDTA) using a 15 mL 30KD ultrafiltration device for three times in total (the final volume was about 1 mL), transferred to a new Eppendorf centrifuge tube (weighed), and weighed. The concentration of the protein was detected and the total amount of the protein was calculated. 2.5 times molar amount of DTT was added to the antibody, the mixture was incubated at room temperature for 2 h and mixed continuously. The mixture was buffer exchanged into a coupling buffer (50 mM Tris, pH 7.2, 150 mM NaCl, 5 mM EDTA) using a 15 ml 30KD ultrafiltration device for three times in total. The concentrated solution was taken, measured for the concentration of the protein by A280, and weighed, and the total amount of the protein was calculated. 10 μl of sample was taken to measure the number of free thiol groups by Ellman's test;

and the molar concentration of its free thiol groups was calculated according to the following formula:

$$C_{thiol}=\frac{A412\times 11.2}{b\times 14150}\ (M)$$

b: optical path length of cuvette (usually 1 cm).

The mole number of free thiol groups was calculated according to the molar concentration of free thiol groups and the volume of the total protein solution.

Vc-MMAE (purchased from Shanghai Haoyuan Chemexpress Co., Ltd., Cat. No. HY-15575) (dissolved in DMSO), 1.1 times the mole number of free thiol groups, was added to the reduced antibody, and the mixture was well mixed to react at room temperature for 2 h, with intermittent mixing. N-acetylcysteine, 20 times the mole number of the vc-MMAE, was added to the reaction system. The reaction mixture was well mixed, and allowed to stand for 5 min. The mixture was buffer exchanged into the conjugation stock solution (20 mM Na-citrate, 0.3% NaCl, 5% Trehalose, 0.05% Tween-80, pH 6.0) using a 15 ml 30KD ultrafiltration device for three times in total to obtain the antibody-drug conjugate MYK-3. The sample was stored at 4 °C. It should be noted that the antibody drug conjugate MYK-3 had been prepared after the conjugation reaction was quenched with N-acetylcysteine. The subsequent step (that is, the mixture was buffer exchanged into the conjugation stock solution using a 15 ml 30KD ultrafiltration device) was performed to place the antibody-drug conjugate MYK-3 in the conjugation stock solution for storage and later use.

Determination of drug-to-antibody ratio:

The prepared antibody-drug conjugate MYK-3 was analyzed by HIC-HPLC (Jun Ouyang, Drug-To-Antibody (DAR) Ratio and Drug Distribution by Hydrophobic Interaction Chromatography and Reverse Phase High Performance Chromatography, Laurent Ducry (ed.), Antibody Drug Conjugates, Chapter 17, Methods in Molecular Biology, Vol 1045, p275-283) to determine the drug-to-antibody ratio (DAR). As shown in Fig. 1, the average DAR calculated according to the peak area of the spectrum was 4.1.

Example 2: Detection of Inhibition Activity of Antibody-Drug Conjugate MYK-3 on Cells in Vitro

Method for detection of inhibition activity on cells:

1.1 After the resuscitated cell lines had been passaged for 3-4 generations, the culture medium was removed first, the cells were then rinsed once with 5 mL of DPBS, digested with 3 mL of trypsin, resuspended in a culture medium and centrifuged with a centrifuge. The supernatant was discarded. The cells were resuspended again with a culture medium. 0.5 mL of cell resuspension was taken to account cells with a cell counter. The cells were plated on 96-well cell culture plates (DiFi cells were plated at 10000 cells/well, HT-29 cells were plated at 5000 cells/well, A549 cells were plated at 2000 cells/well, U87-MG cells were plated at 3000 cells/well, and LoVo cells were plate at 4000 cells/well). After 24 h of culture, the monoclonal antibody BA03 and the antibody-drug conjugate MYK-3 as diluted in a series of concentrations were added and incubated for 72 h. Then, 20 μl of CCK8 color reagent was added to each well, OD450-650 was tested at a wavelength of 450-650 nm on a microplate reader, and four-parameter fitting was performed.

Results of the detection of inhibition activity on cells in vitro:

The following cell lines were purchased from Shanghai Institute of Cell Biology, Chinese Academy of Sciences.

Activity in DiFi cells (human colorectal cancer cells) with high EGFR expression: MYK-3 had significantly higher

cell growth inhibition activity than the monoclonal antibody BA03, and the $EC_{50}$ was reduced by about 10 times ($EC_{50}$ of BA03 was 51.9 ng/ml, and $EC_{50}$ of MYK-3 was 5.1 ng/ml), as shown in Fig. 2.

Activity in other tumor cells with moderate and low expression of EGFR: compared with the monoclonal antibody itself, MYK-3 also showed obvious cell growth inhibition activity against cancer cells (human colon cancer cell HT29, human lung cancer cell A549, human brain astroblastoma cell U87-MG) with moderate and low expression of EGFR (as shown in Fig. 3, Fig. 4, and Fig. 5), where $EC_{50}$ of HT-29 was 611 ng/ml, $EC_{50}$ of A549 was 28.3 μg/ml, and $EC_{50}$ of U87-MG was 5.3 μg/ml.

In addition, we also tested its activity in LoVo, a KRAS mutant colon cancer cell with moderate expression of EGFR (Dunn EF, Ilda M, Myers RA, Hintz KA, Campbell DA, Armstrong EA, Li C and Wheeler DL. Dasatinib sensitizes KRAS mutant colorectal tumors to cetruximab. Oncogene 2011; 30: 561-574), and found that MYK-3 showed significant tumor growth inhibition activity against the KRAS mutant colon cancer cell LoVo (as shown in Fig. 6, $EC_{50}$ was 3.2 μg/ml), but BA03 had almost no inhibitory activity against that cell line when used alone.

Example 3: In Vivo tumor xenograft test in mice

Experimental Method of In Vivo tumor xenograft test in mice:

HT-29 colon cancer cells were cell lines with relatively low expression of EGFR and with BRAF mutations, and the EGFR targeting monoclonal antibody Erbitux as currently marketed for the treatment of colorectal cancer had no growth inhibition activity against HT-29 cell lines.

HT-29 cell xenograft model: the tumor cells at logarithmic growth phase were collected and counted, then resuspended in 1×PBS. The cell suspension concentration was adjusted to $3\times10^7$/ml. The tumor cells were inoculated subcutaneously on the right side of back of nude mice with a 1 ml syringe (4 gauge needle), $3 \times 10^6$/0.1ml/mouse. When the tumor volume reached 150-200 $mm^3$, the mice were grouped by a randomized block method, 8 mice per group, so as to ensure that the tumor volume and body weight of mice between the groups were uniform. The difference between the mean value of tumor volume in each group and the mean value of tumor volume of all experimental animals was not more than ± 10%. Tail vein administration was performed, once every four days (the $1^{st}$, $5^{th}$, $9^{th}$ and $13^{th}$ day), for a total of 4 times, and the tumor volumes and body weights of mice were regularly measured. There were 8 mice in each administration group.

Experimental results of tumor xenograft study in mice

HT-29 colon cancer xenograft test in mice: there were 5 groups in the test, including buffer solution group (20 mM sodium citrate, 0.3% sodium chloride, 5% trehalose, 0.05% Tween 80, pH 6), BA03 monoclonal antibody group (5 mg/kg), MYK-3 group (1 mg/kg), MYK-3 group (5 mg/kg) and non-binding ADC group (5 mg/kg) (human IgG-vcMMAE conjugate, in which IgG was IgG obtained by purification from human serum, and this conjugate was prepared by the same method as MYK-3). The xenograft tumor volume in the mice administrated with MYK-3 was significantly lower than that of the control group, showing a significant anti-tumor growth effect (Fig. 7). On the $18^{th}$ day, for the group of MYK-3 at dose of 5 mg/Kg, its tumor growth inhibition rate was up to 54% as compared with the buffer solution group, and its tumor growth inhibition rate was up to 46% as compared with the group of monoclonal antibody BA03 at the same dose, and its tumor growth inhibition rate was up to 42% as compared with the non-binding ADC.

Body weight of mice: the body weight of mice administrated with MYK-3 showed no significant change as compared with the control group (see Fig. 8), indicating that MYK-3 had not toxic effect of reducing body weight of mice.

Example 4: Growth inhibition activity of MYK-3 against xenograft tumors with KRAS mutant colon cancer cell LoVo in nude mice

According to the model construction (inoculated with the tumor cell at $2\times10^6$ cells/0.1ml/mouse) and administration method of Example 3, the growth inhibition activity of MYK-3 on the xenograft tumors with KRAS mutant colon cancer cell LoVo in nude mice was tested. The experiment was performed in six groups, including a dilution buffer solution group (20 mM Na-citrate, 0.3% NaCl, 5% Trehalose, 0.05% Tween 80, pH 6), an Erbitux monoclonal antibody group (3 mg/kg), MYK-3 groups (with three doses of 0.3 mg/kg, 1 mg/kg, and 3 mg/kg) and a non-binding ADC control group (3 mg/kg), wherein the non-binding control ADC represented non-binding ADC control (which was anti-CD20 mAb-vcMMAE) with the same loading. The preparation method of the conjugate was the same as that of MYK-3. The experimental results are shown in Fig. 9.

As can be seen from the figure, MYK-3 showed complete inhibition on the growth of LoVo cell tumor at a dose of 3 mg/Kg, and MYK-3 had shown higher activity at a dose of 1 mg/Kg than marketed drug Erbitux at a dose of 3 mg/Kg.

Example 5 Effect of pH on the stability of MYK-3

Using the antibody-drug conjugate obtained in Example 1 as a starting point, the inventor conducted a more in-depth study on pH. The results showed that the pH of the optimal MYK-3 formulation is 6.3. That is, the optimal formulation includes 20 mM citrate buffer, 5.5% trehalose dihydrate, 0.3% NaCl and 0.05% PS80, pH 6.3, and the concentration of the antibody-drug conjugate MYK-3 is 4 mg/mL.

In order to investigate the effect of different pH on the quality of MYK-3 in 20 mM citrate buffer (containing 5.5% trehalose dihydrate, 0.3% NaCl and 0.05% PS80), the same procedure as in Example 1 was conducted except the formulation (Formulations F1-F5 to be investigated) of the conjugation stock solution into which the conjugation reaction product was buffer exchanged using a 15 ml 30KD ultrafiltration device after the conjugation reaction was quenched with N-acetylcysteine.

Formulations F1-F5 to be investigated were the same in composition and concentration except for the pH of the citrate buffer. Specifically: 4 mg/mL antibody-drug conjugate MYK-3, 20 mM citrate buffer, pH 5.6 (F1), 6.0 (F2), 6.3 (F3), 6.5 (F4) or 6.7 (F5), 5.5% trehalose dihydrate, 0.3% NaCl and 0.05% PS80.

Specifically, the conjugation reaction product was exchanged into the Formulations F1-F5 (pH: 5.6-6.7) to be investigated and then investigated under 40±2°C/75%±5%RH for 10 days, and samples were taken for analysis at set time points. The test items included appearance, pH, protein concentration, SEC, iCIEF and HIC. If the sample appears turbid or has obvious opalescence, subsequent analysis will not be performed (such as SEC, iCIEF and HIC). The specific experimental design is shown in Table 1.

Table 1 Experimental design of the effect of pH (5.6-6.7) on the stability of MYK-3 (in 20 mM citrate buffer)

| Formulation | pH | T0 | 40±2°C/75%±5%RH | |
|---|---|---|---|---|
| | | | 3D | 7D |
| F1 | 5.6 | √ | √ | √ |
| F2 | 6.0 | √ | √ | √ |
| F3 | 6.3 | √ | √ | √ |
| F4 | 6.5 | √ | √ | √ |
| F5 | 6.7 | √ | √ | √ |
| Note: T0 represents the starting point, D represents day, and √ represents test. | | | | |

In terms of appearance, as time increased, the samples of F1 placed at 40 °C for 3 days became turbid, the samples of F2 placed at 40 °C for 7 days became turbid, obvious opalescence and precipitation were observed in samples of F1 and F2 when the temperature was reduced to room temperature and 2-8 °C, and the lower the pH, the more precipitation occurred; however, samples of other formulations always remained clear and no significant changes were observed.

Test data of SEC, iCIEF and HIC and changing trends are shown in Table 2 and Fig. 10. Since the samples of F1 placed at 40 °C for 3 days became turbid, the samples of the formulation F1 were not tested by SEC, iCIEF and HIC on day 3 and day 7. And since the samples of F2 placed at 40 °C for 7 days became turbid, the samples of F2 were not tested by SEC, iCIEF and HIC on day 7. HIC data shows that the DAR of F3 and F4 are basically unchanged with different pH values (Fig. 10c); SEC data shows that with the increase of pH, the growth rate of HMW% of F3, F4 and F5 shows a downward trend and their downward trends are basically the same (Fig. 10a); iCIEF data shows that with the increase of pH, the acidic peak % of F3, F4 and F5 shows an upward trend but the upward trend of F3 is obviously lower than the upward trends of F4 and F5 (Fig. 10b). Based on the above test data and in consideration of test results about appearance, SEC, iCIEF, HIC and the like, it is determined that the optimal formulation of MYK-3 is F3, that is, the pH of the formulation is 6.3. Accordingly, the amount of citric acid and sodium citrate in the formulations were adjusted, and the final formulation gives a 20 mM citrate buffer with pH 6.3.

Table 2 Experimental data of the effect of pH (5.6-6.7) on the stability of MYK-3 (in 20 mM citrate buffer)

| Formulation | | SEC | | | iCIEF | | | HIC |
|---|---|---|---|---|---|---|---|---|
| | | HMW (%) | Monomer (%) | LMW (%) | Acidic peak (%) | Primary peak (%) | Alkaline peak (%) | DAR |
| pH 5.6 | 0d | 2.2 | 97.8 | 0.1 | 23.3 | 49.5 | 27.3 | 4.0 |
| | 3d | Samples became turbid | | | | | | |
| pH 6.0 | 0d | 2.3 | 97.6 | 0.1 | 23.0 | 50.7 | 26.3 | 4.0 |
| | 3d | 11.9 | 88.0 | 0.1 | 24.9 | 50.7 | 24.5 | 4.1 |
| | 7d | Samples became turbid | | | | | | |
| pH 6.3 | 0d | 2.3 | 97.7 | 0.1 | 24.0 | 48.0 | 27.9 | 4.0 |
| | 3d | 8.6 | 91.3 | 0.1 | 29.9 | 51.6 | 18.5 | 4.0 |
| | 7d | 10.8 | 89.1 | 0.1 | 34.0 | 46.7 | 19.2 | 4.0 |
| pH 6.5 | 0d | 2.3 | 97.7 | 0.1 | Not tested | | | 4.0 |
| | 3d | 8.6 | 91.2 | 0.1 | 32.0 | 51.5 | 16.5 | 4.0 |
| | 7d | 9.9 | 90.1 | 0.1 | 42.0 | 44.2 | 13.8 | 4.0 |
| pH 6.7 | 0d | 2.3 | 97.7 | 0.1 | 21.2 | 52.2 | 26.5 | 4.0 |
| | 3d | 8.9 | 91.0 | 0.1 | 35.7 | 45.7 | 18.6 | 4.0 |
| | 7d | 9.8 | 90.1 | 0.1 | 48.1 | 39.4 | 12.5 | 3.9 |

Based on the above data the final MYK-3 formulation was as follows:

| Adjuvant | Purpose | Amount (g/L) | Manufacturer | Quality standard |
|---|---|---|---|---|
| Citric acid monohydrate | pH adjuster | 0.27 | Merck Chemical Technology (Shanghai) Co., Ltd. | ChP |
| Sodium citrate dihydrate | pH adjuster | 5.50 | J.T Baker | USP |
| Trehalose dihydrate | Protein protectant | 55.26 | Pfanstiehl | ChP |
| PS80 | Antitack agent, protein protectant | 0.50 | Nanjing Well Chemical Co., Ltd. | ChP |
| NaCl | Salt | 3.00 | Jiangsu Province Qinfen Pharmaceutical Co., Ltd. | ChP |

Although the specific embodiments of the invention have been described in detail, those skilled in the art will understand that modifications and substitutions can be made to the details according to all the teachings that have been disclosed, and these changes are within the scope of the invention. The full scope of the invention is given by the appended claims and any equivalents thereof.

## Claims

**1.** An antibody-drug conjugate formulation, comprising:

an antibody-drug conjugate or a salt thereof with a concentration of 1-20 mg/mL, preferably 2-6 mg/mL, more preferably 4 mg/mL;
a citrate buffer with a concentration of 10-50 mM, preferably 15-25 mM, more preferably 20 mM, and a pH of

6.3-6.7, preferably 6.3-6.5, more preferably 6.3;
trehalose with a concentration of 1-10%, preferably 4-7%, more preferably 5.5%;
NaCl with a concentration of 0.1-2%, preferably 0.1-0.5%, more preferably 0.3%;
Tween 80 with a concentration of 0.01-0.2%, preferably 0.01-0.1%, and more preferably 0.05%;
wherein the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID Nos: 5-7 or mutants thereof, and CDR1, CDR2, and CDR3 in a light chain variable region respectively comprise sequences as shown in SEQ ID Nos: 12-14 or mutants thereof;
L represents a linker, and the linker is 6-maleimidocaproyl-valine-citrulline-p-aminobenzyloxycarbonyl (MC-vc-PAB);
D represents a cytotoxic agent, and the cytotoxic agent is MMAE; and
p represents 1-9, preferably 2-6, more preferably 3-5.

**2.** The antibody-drug conjugate formulation according to claim 1, wherein the anti-EGFR antibody has one or more of the following features:

i. FR1, FR2, FR3, and FR4 regions in the heavy chain variable region of the anti-EGFR antibody respectively comprise sequences as shown in SEQ ID Nos: 8-11 or mutants thereof;
ii. FR1, FR2, FR3, and FR4 regions in the light chain variable region of the anti-EGFR antibody respectively comprise sequences as shown in SEQ ID Nos: 15-18 or mutants thereof;
iii. the heavy chain constant region of the anti-EGFR antibody is selected from human IgG, IgM, IgA, IgD, and IgA constant regions or mutants thereof;
iv. preferably, the IgG is selected from IgG1, IgG2, IgG3 and IgG4; and
v. the light chain constant region of the anti-EGFR antibody is selected from human lambda and kappa constant regions or mutants thereof.

**3.** The antibody-drug conjugate formulation according to any one of claims 1-2, wherein the anti-EGFR antibody has one or more of the following features:

i. the sequence of the heavy chain variable region of the anti-EGFR antibody comprises a sequence as shown in SEQ ID NO: 1, or a sequence having greater than 70%, preferably greater than 75%, 80%, 85%, 90%, 95%, or 99% identity to the sequence as shown in SEQ ID NO: 1;
preferably, the sequence of the heavy chain variable region of the anti-EGFR antibody is shown in SEQ ID NO: 1;
ii. the sequence of the light chain variable region of the anti-EGFR antibody comprises a sequence as shown in SEQ ID NO: 2, or a sequence having greater than 70%, preferably greater than 75%, 80%, 85%, 90%, 95%, or 99% identity to the sequence as shown in SEQ ID NO: 2;
preferably, the sequence of the light chain variable region of the anti-EGFR antibody is shown in SEQ ID NO: 2;
iii. the sequence of the heavy chain constant region of the anti-EGFR antibody comprises a sequence as shown in SEQ ID NO: 3, or a sequence having greater than 70%, preferably greater than 75%, 80%, 85%, 90%, 95%, or 99% identity to the sequence as shown in SEQ ID NO: 3;
preferably, the sequence of the heavy chain constant region of the anti-EGFR antibody is shown in SEQ ID NO: 3;
iv. the sequence of the light chain constant region of the anti-EGFR antibody comprises a sequence as shown in SEQ ID NO: 4, or a sequence having greater than 70%, preferably greater than 75%, 80%, 85%, 90%, 95%, or 99% identity to the sequence as shown in SEQ ID NO: 4; and
preferably, the sequence of the light chain constant region of the anti-EGFR antibody is shown in SEQ ID NO: 4.

**4.** The antibody-drug conjugate formulation according to any one of claims 1-3, wherein the trehalose is trehalose dihydrate;

or, the citrate buffer is prepared from citric acid and sodium citrate;
preferably, the citric acid is citric acid monohydrate;
preferably, the sodium citrate is sodium citrate dihydrate.

5. A method for preparing the formulation according to any one of claims 1-4, comprising:

i. allowing the anti-EGFR antibody as defined in any one of claims 1-3 to have a reduction reaction with a reducing agent to obtain a reduced anti-EGFR antibody;
ii. allowing the reduced anti-EGFR antibody to have a conjugation reaction with vcMMAE; and
iii. quenching the conjugation reaction, and performing buffer exchange of the conjugation reaction product to obtain the antibody-drug conjugate formulation as defined in any one of claims 1-4.

6. The method according to claim 5, comprising:

i. exchanging the anti-EGFR antibody (preferably three times) as defined in any one of claims 1-3 (preferably 10 mg) into a reducing buffer (preferably comprising: 25 mM sodium borate, pH 8.0, 25 mM NaCl, 5 mM EDTA), detecting the concentration of the protein, and calculating the amount of the protein;
ii. adding DTT to the product of step (1) to have a reaction at room temperature for 1-5 h (preferably 2 h) to obtain a reduced anti-EGFR antibody, wherein the amount of substance of the DTT is 2.0-3.0 times, preferably 2.5 times, the amount of substance of the protein;
iii. exchanging the product of step (2) (preferably three times) into a coupling buffer (preferably comprising: 50 mM Tris, pH 7.2, 150 mM NaCl, 5 mM EDTA), and calculating the amount of substance of free thiol groups;
iv. adding vc-MMAE to the product of step (3) to have a reaction at room temperature for 1-5 h (preferably 2 h), wherein the amount of substance of the vc-MMAE is 1.0-1.5 times, preferably 1.1 times, the amount of substance of the reduced anti-EGFR antibody;
v. adding N-acetylcysteine to the product of step (4) and allowing to stand (preferably for 5 min) to obtain a mixed solution containing the antibody-drug conjugate as defined in claim 1, wherein the amount of substance of the N-acetylcysteine is 15-25 times, preferably 20 times, the amount of substance of the vc-MMAE;
vi. exchanging the mixed solution of step (5) (preferably three times) into the conjugation stock solution to obtain the antibody-drug conjugate formulation;

the conjugation stock solution comprises:

a citrate buffer with a concentration of 10-50 mM, preferably 15-25 mM, more preferably 20 mM and pH of 6.3-6.7, preferably 6.3-6.5, more preferably 6.3;
trehalose with a concentration of 1-10%, preferably 4-7%, more preferably 5.5%;
NaCl with a concentration of 0.1-2%, preferably 0.1-0.5%, more preferably 0.3%; and
Tween 80 with a concentration of 0.01-0.2%, preferably 0.01-0.1%, more preferably 0.05%;
in the antibody-drug conjugate formulation, the concentration of the antibody-drug conjugate is 1-20 mg/mL, preferably 2-6 mg/mL, more preferably 4 mg/mL;
preferably, the trehalose is trehalose dihydrate;
preferably, the citrate buffer is prepared from citric acid and sodium citrate;
preferably, the citric acid is citric acid monohydrate;
preferably, the sodium citrate is sodium citrate dihydrate.

7. An antibody-drug conjugate formulation prepared by the method according to any one of claims 5-6.

8. A composition, comprising the formulation according to any one of claims 1-4 and 7, optionally further comprising a pharmaceutically acceptable carrier, a diluent or an excipient.

9. A formulation according to any one of claims 1-4 and 7 or the composition according to claim 8:

i. for preventing and/or treating an EGFR-related disease; and/or
ii. for inhibiting tumor angiogenesis, delaying tumor progression, inhibiting tumor growth, or inhibiting tumor cell proliferation.

10. The formulation for use according to claim 9, wherein

the EGFR-related disease is an EGFR-related tumor, such as a tumor related to EGFR overexpression, further, for example, selected from the group consisting of colon cancer, rectal cancer, head and neck cancer, lung cancer, ovarian cancer, cervical cancer, bladder cancer, esophageal cancer, breast cancer, kidney cancer, prostate cancer, stomach cancer, pancreatic cancer and brain glioma;

or, the tumor is selected from the group consisting of colon cancer, rectal cancer, head and neck cancer, lung cancer, ovarian cancer, cervical cancer, bladder cancer, esophageal cancer, breast cancer, kidney cancer, prostate cancer, stomach cancer, pancreatic cancer and brain glioma;
or, the tumor is a tumor with KRAS gene mutation, further, for example, colon cancer, rectal cancer, lung cancer or pancreatic cancer with KRAS gene mutation;
or, the tumor is a tumor with BRAF gene mutation, further, for example, selected from the group consisting of colon cancer, rectal cancer and lung cancer with BRAF gene mutation.

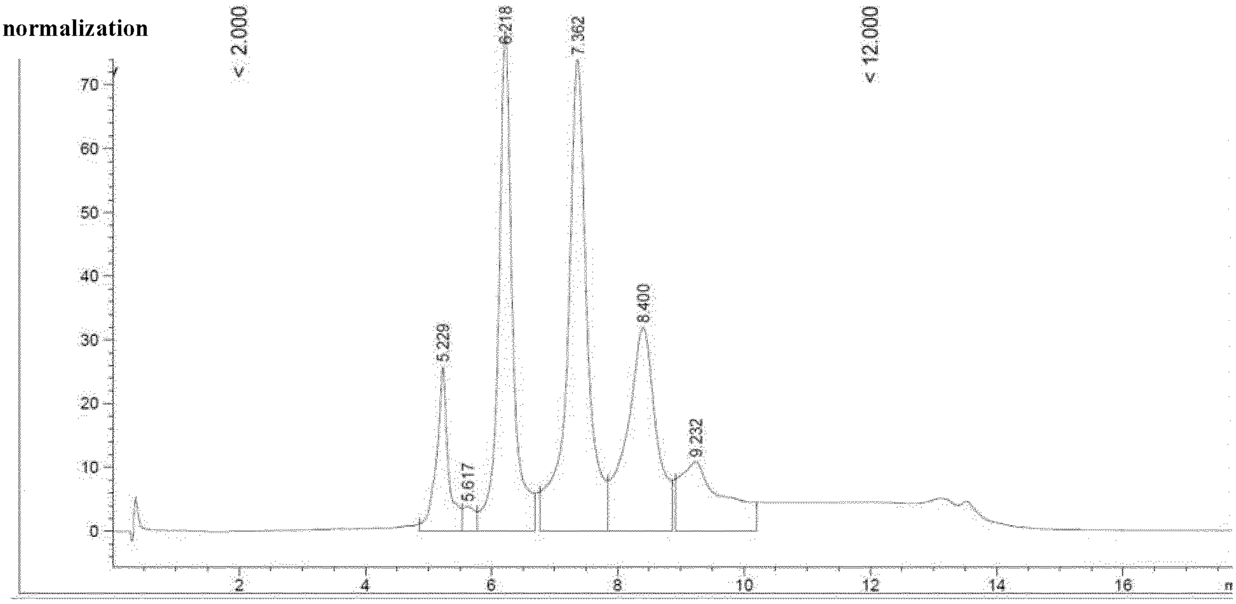
normalization
< 2.000
6.218
7.362
< 12.000
8.400
5.229
9.232
5.617
70
60
50
40
30
20
10
0
2
4
6
8
10
12
14
16

Fig. 1

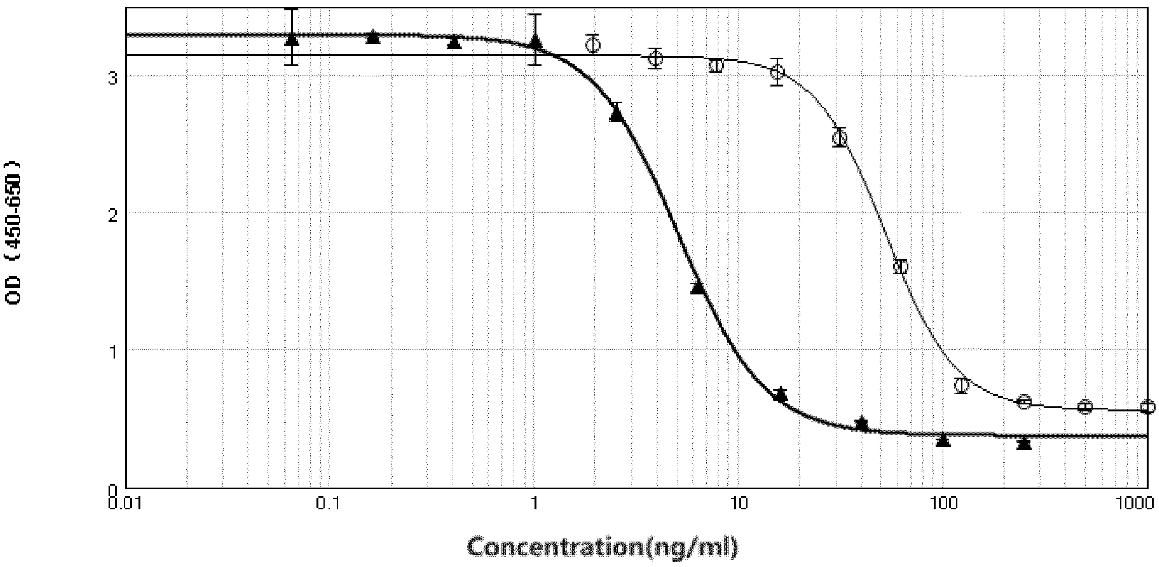
OD（450-650）
3
2
1
0
0.01
0.1
1
10
100
1000
Concentration(ng/ml)

Fig. 2

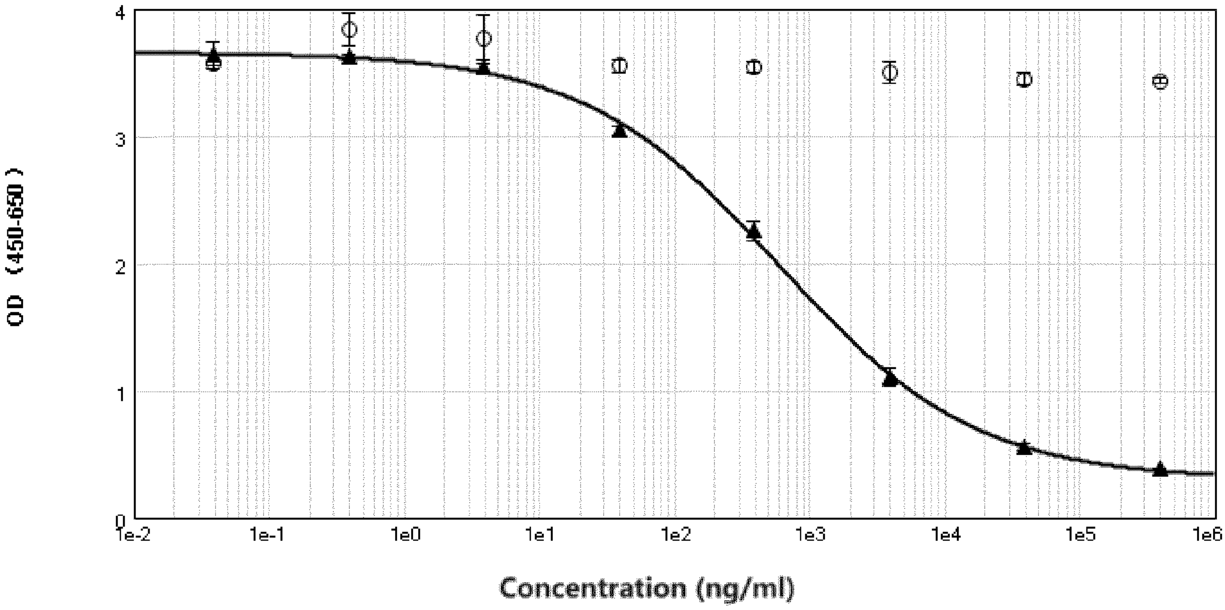
OD （450-650）
4
3
2
1
0
1e-2
1e-1
1e0
1e1
1e2
1e3
1e4
1e5
1e6
Concentration (ng/ml)

Fig. 3

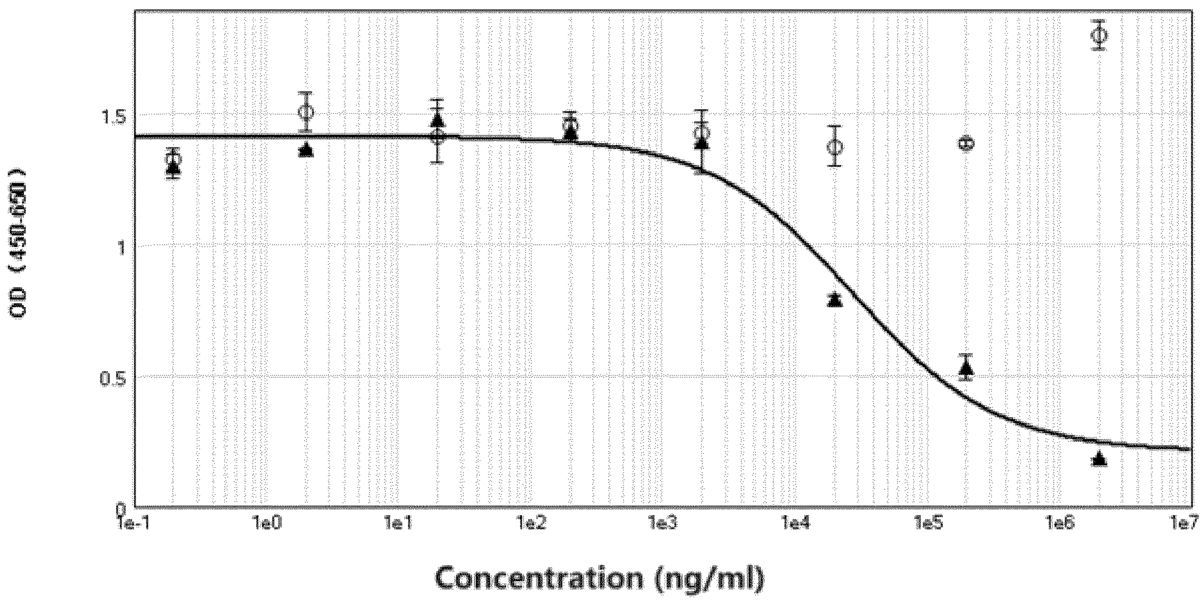
OD（450-650）
1.5
1
0.5
0
1e-1
1e0
1e1
1e2
1e3
1e4
1e5
1e6
1e7
Concentration (ng/ml)

Fig. 4

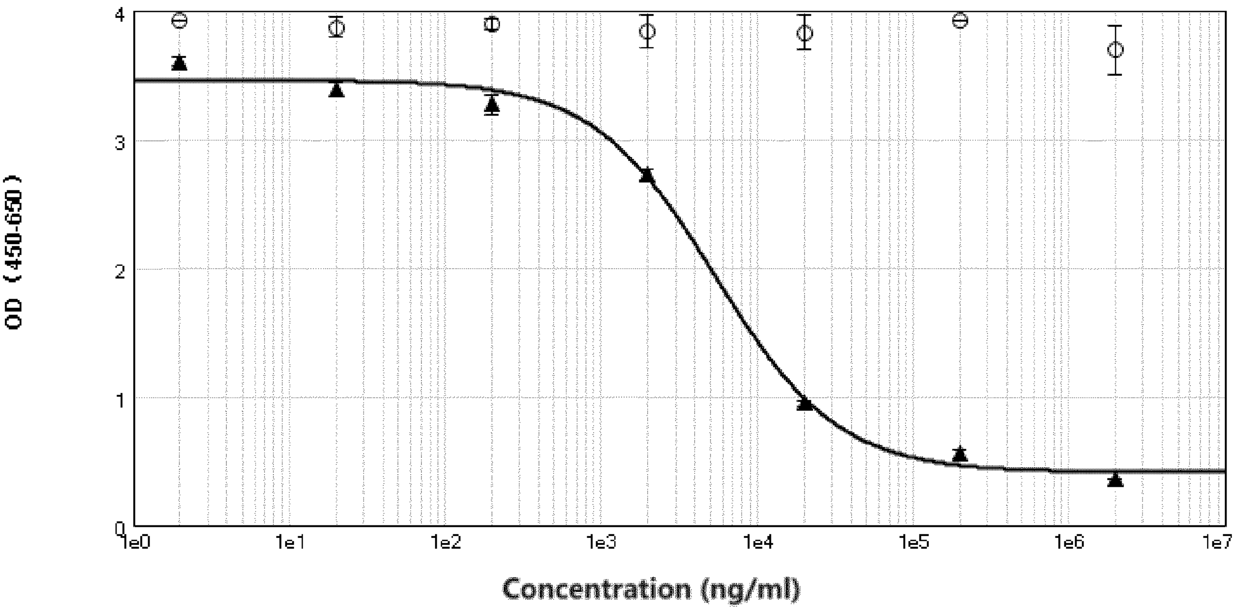
OD（450-650）
0
1
2
3
4
1e0
1e1
1e2
1e3
1e4
1e5
1e6
1e7
Concentration (ng/ml)

Fig. 5

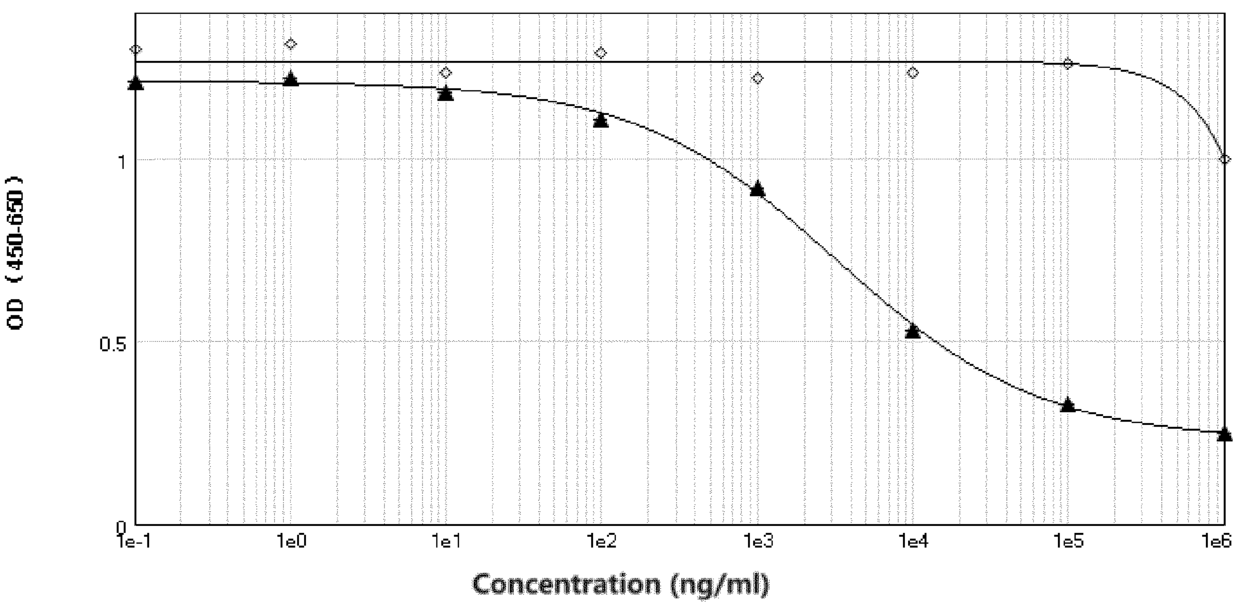
OD（450-650）
0
0.5
1
1e-1
1e0
1e1
1e2
1e3
1e4
1e5
1e6
Concentration (ng/ml)

Fig. 6

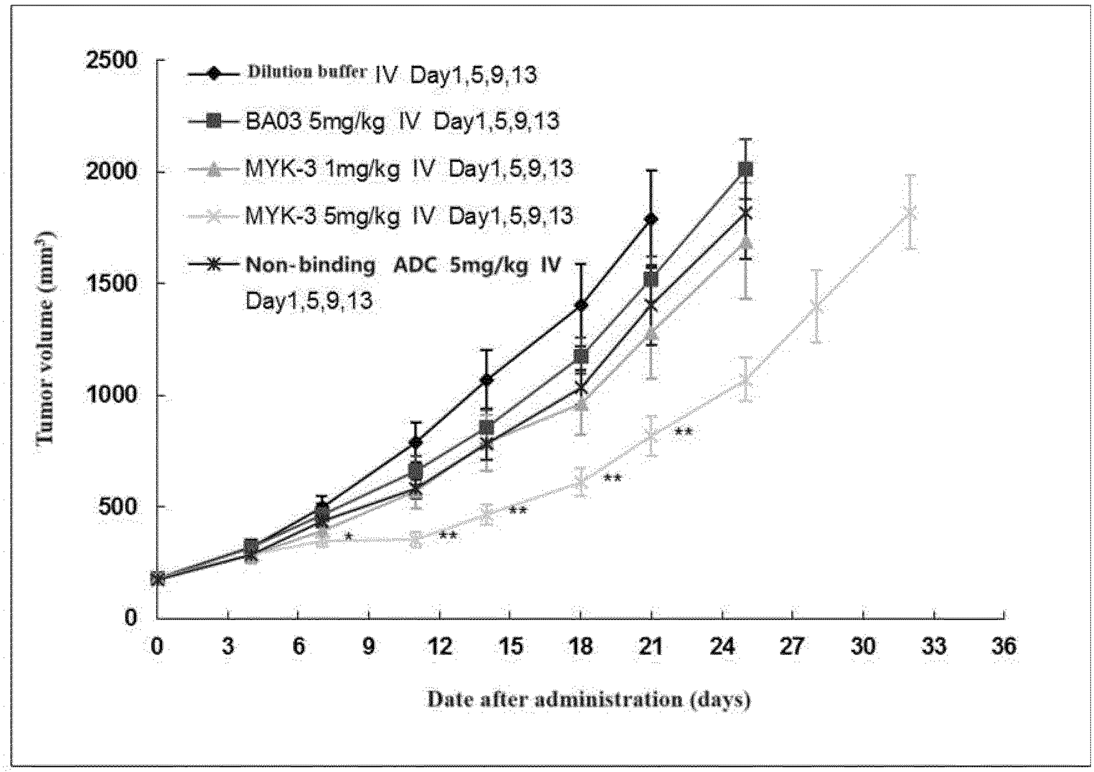
Dilution buffer IV Day1,5,9,13
BA03 5mg/kg IV Day1,5,9,13
MYK-3 1mg/kg IV Day1,5,9,13
MYK-3 5mg/kg IV Day1,5,9,13
Non-binding ADC 5mg/kg IV Day1,5,9,13
Tumor volume (mm³)
Date after administration (days)

Fig. 7

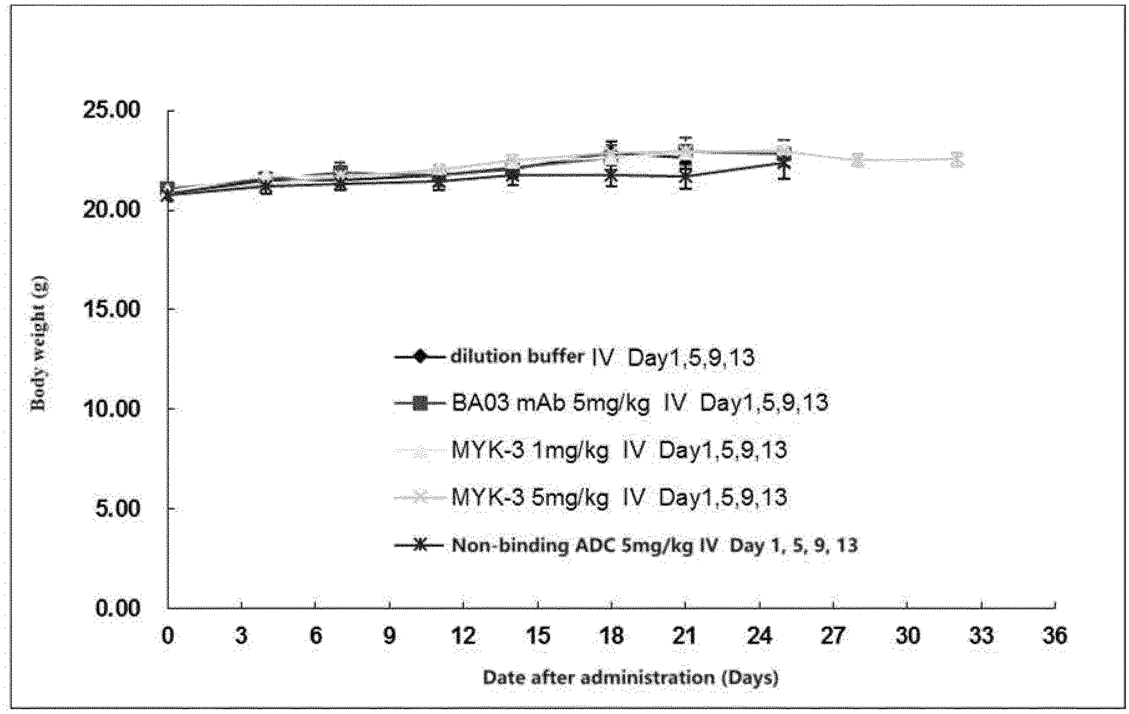
dilution buffer IV Day1,5,9,13
BA03 mAb 5mg/kg IV Day1,5,9,13
MYK-3 1mg/kg IV Day1,5,9,13
MYK-3 5mg/kg IV Day1,5,9,13
Non-binding ADC 5mg/kg IV Day 1, 5, 9, 13
Body weight (g)
Date after administration (Days)

Fig. 8

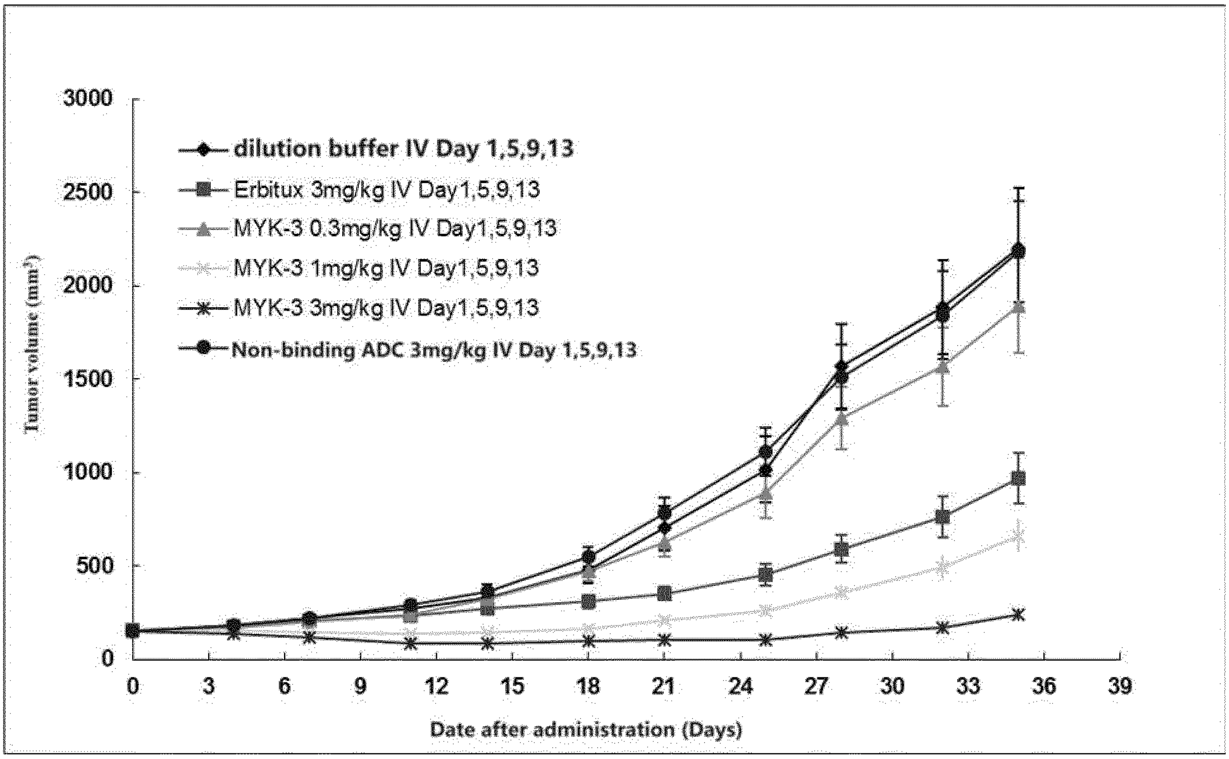
3000
2500
2000
1500
1000
500
0
Tumor volume (mm³)
dilution buffer IV Day 1,5,9,13
Erbitux 3mg/kg IV Day1,5,9,13
MYK-3 0.3mg/kg IV Day1,5,9,13
MYK-3 1mg/kg IV Day1,5,9,13
MYK-3 3mg/kg IV Day1,5,9,13
Non-binding ADC 3mg/kg IV Day 1,5,9,13
0
3
6
9
12
15
18
21
24
27
30
33
36
39
Date after administration (Days)

Fig. 9

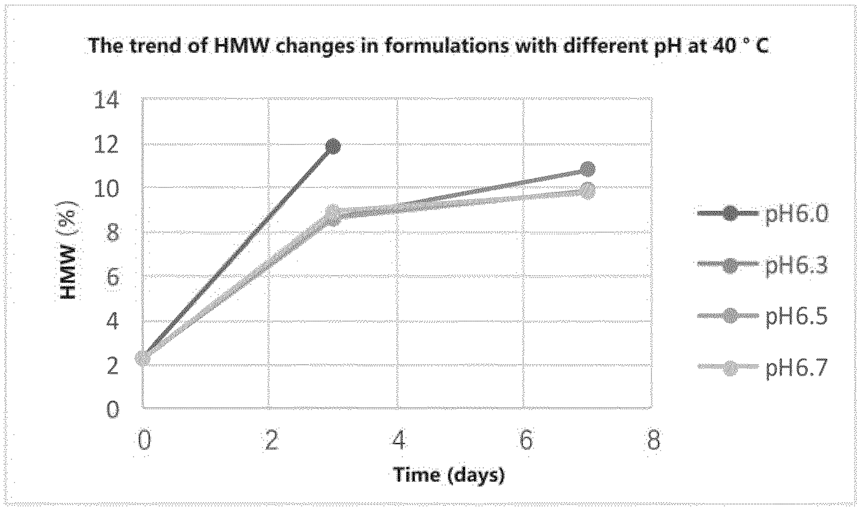
The trend of HMW changes in formulations with different pH at 40 ° C
HMW (%)
Time (days)
pH6.0
pH6.3
pH6.5
pH6.7

a

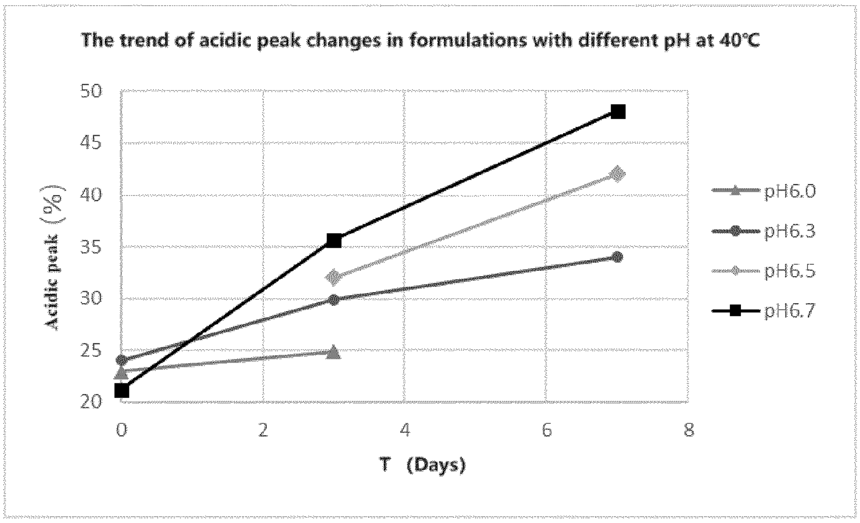
The trend of acidic peak changes in formulations with different pH at 40℃
Acidic peak (%)
T (Days)
pH6.0
pH6.3
pH6.5
pH6.7

b

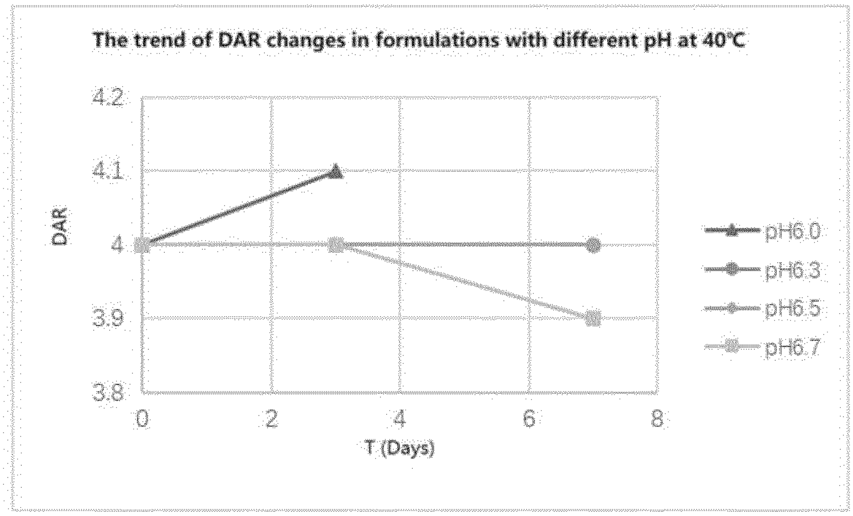
The trend of DAR changes in formulations with different pH at 40℃
DAR
T (Days)
pH6.0
pH6.3
pH6.5
pH6.7

c

Fig. 10

**INTERNATIONAL SEARCH REPORT**

International application No. **PCT/CN2021/118656**

**A. CLASSIFICATION OF SUBJECT MATTER**

A61K 47/68(2017.01)i；A61K 45/00(2006.01)i；A61P 35/00(2006.01)i

According to International Patent Classification (IPC) or to both national classification and IPC

**B. FIELDS SEARCHED**

Minimum documentation searched (classification system followed by classification symbols)

A61K，A61P

Documentation searched other than minimum documentation to the extent that such documents are included in the fields searched

Electronic data base consulted during the international search (name of data base and, where practicable, search terms used)

CNABS, CNTXT, DWPI, SIPOABS, CATXT, USTXT, EPTXT, WOTXT, KRTXT, JPTXT, CPEA, CNKI, WEB OF SCIENCE, PUBMED, 百度学术搜索, BAIDU SCHOLAR: 抗体, 单克隆, 单抗, 表皮生长因子受体, EGFR, 偶联, ADC, 柠檬酸, 海藻糖, 氯化钠, 吐温80, antibody, monoclonal, mAb, Epidermal Growth Factor Receptor, coupled, citric acid, trehalose, sodium chloride, Tween 80, Polysorbate 80 GenBank, EMBL, 中国专利生物序列检索系统, China Patents Biological Sequence Search System: 对SEQ ID NOs: 1-18的序列检索, sequence search for SEQ ID NOs: 1-18

**C. DOCUMENTS CONSIDERED TO BE RELEVANT**

| Category* | Citation of document, with indication, where appropriate, of the relevant passages | Relevant to claim No. |
|---|---|---|
| X | CN 111529717 A (SHANGHAI MIRACOGEN INC.) 14 August 2020 (2020-08-14)<br>see claims 1-9, 14, 17, and 20-25, and description, paragraphs 148 and 161-167 | 1-10 |
| Y | CN 107773755 A (SHANGHAI JMT-BIO INC.) 09 March 2018 (2018-03-09)<br>see claims 1 and 7-8, and description, paragraph 93 | 1-10 |
| Y | CN 109200291 A (INSTITUTE OF MEDICINAL BIOTECHNOLOGY, CHINESE ACADEMY OF MEDICAL SCIENCES) 15 January 2019 (2019-01-15)<br>see claims 1-3 | 1-10 |
| Y | CN 103772504 A (SHANGHAI JMT-BIO INC.) 07 May 2014 (2014-05-07)<br>see description, paragraph 123 | 1-10 |
| A | WO 2020173431 A2 (INNO-N INNOVENT BIOLOGICS SUZHOU CO., LTD.) 03 September 2020 (2020-09-03)<br>see entire document | 1-10 |

☐ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 November 2021** | **10 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/118656**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2021/118656**

| Patent document cited in search report | Publication date (day/month/year) | Patent family member(s) | Publication date (day/month/year) |
|---|---|---|---|
| CN 111529717 A | 14 August 2020 | WO 2016131409 A1 | 25 August 2016 |
| | | US 2018036423 A1 | 08 February 2018 |
| | | US 10792370 B2 | 06 October 2020 |
| | | CN 106999606 A | 01 August 2017 |
| CN 107773755 A | 09 March 2018 | None | |
| CN 109200291 A | 15 January 2019 | None | |
| CN 103772504 A | 07 May 2014 | None | |
| WO 2020173431 A2 | 03 September 2020 | CA 3131307 A1 | 03 September 2020 |
| | | AU 2020227770 A1 | 23 September 2021 |
| | | WO 2020173431 A3 | 22 October 2020 |
| | | TW 202045136 A | 16 December 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103772504 A


### Non-patent literature cited in the description


- **ALLEGRA CJ ; JESSUP JM ; SOMERIELD MR ; HAMILTON SR ; HAMMOND EH ; HAYES DF et al.** American Society of Clinical Oncology provisional clinical opinion: testing for KRAS gene mutations in patients with metastatic colorectal carcinoma to predict response to anti-epidermal growth factor monoclonal antibody therapy. *J. Clin Oncol.,* 2009, vol. 27, 2091-2096
- **BARDELFI A ; SIENA S.** Molecular mechanisms of resistance to cetuximab and panitumumab in colorectal cancer. *J Clin Oncol.,* 2010, vol. 28, 1254-1261
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **ALTSCHUL et al.** *Nucl. Acid. Res.,* 1977, vol. 25, 3389-3402
- **ALTSCHUL et al.** *J. Mol. Biol.,* 1990, vol. 215, 403-410
- Immunology-A Synthesis. Sinauer Associates, 1991
- Drug-To-Antibody (DAR) Ratio and Drug Distribution by Hydrophobic Interaction Chromatography and Reverse Phase High Performance Chromatography. **JUN OUYANG.** Methods in Molecular Biology. vol. 1045, 275-283
- **DUNN EF ; ILDA M ; MYERS RA ; HINTZ KA ; CAMPBELL DA ; ARMSTRONG EA ; LI C ; WHEELER DL.** Dasatinib sensitizes KRAS mutant colorectal tumors to cetruximab. *Oncogene,* 2011, vol. 30, 561-574